# EUROPEAN PATENT SPECIFICATION

(11) **EP 1 025 226 B1**
(45) Date of publication and mention of the grant of the patent: **06.12.2006**
(21) Application number: 98951141.5
(22) Date of filing: 29.10.1998
(51) Int. Cl.: C12N 15/12, C12N 5/10, C07K 14/705, G01N 33/566, A61K 38/17

(54) **DNA ENCODING A HUMAN PROTON-GATED ION CHANNEL AND USES THEREOF**
DNA KODIEREND FÜR EINEN HUMANEN PROTONENABHÄNGIGEN IONENKANAL UND DEREN VERWENDUNGEN
ADN CODANT UN CANAL IONIQUE HUMAIN A PORTE PROTONIQUE ET UTILISATIONS DE CE DERNIER

(30) Priority: 29.10.1997 CA 2219713
(43) Date of publication of application: 09.08.2000
(73) Proprietor: McGill University, Montreal, Quebec H3A 2A7 (CA)
(72) Inventor: SEGUELA, Philippe, Outremont, Québec H2V 4B5 (CA); BABINSKI, Kazimierz, Dorval, Québec H9S 1H4 (CA)
(74) Representative: MacLean, Martin Robert
(86) International application number: PCT/CA1998/001016
(87) International publication number: WO 1999/021981

(56) References cited:
- WO-A-97/01577
- WO-A-98/35034
- WO-A-98/54316
- WALDMANN R ET AL: "Molecular cloning of a non-inactivating proton-gated Na+ channel specific for sensory neurons" JOURNAL OF BIOLOGICAL CHEMISTRY, vol. 272, no. 34, 22 August 1997, pages 20975-20978, XP002080467 cited in the application
- LEWIS C. ET AL.: "Coexpression of P2X2 and P2X3 receptor subunits can account for ATP-gated currents in sensory neurons." NATURE, vol. 377, 5 October 1995, pages 432-435, XP002098923
- COREY D P ET AL: "MECHANOSENSATION AND THE DEG/ENAC ION CHANNELS" SCIENCE, vol. 273, no. 5273, 19 July 1996, page 323/324 XP002051360 cited in the application
- DATABASE GENBANK Accession No. AF057711, 7 October 1998 BABINSKI K. ET AL.: "H. sapiens proton-gated cation channel subunit (ASIC3)" XP002098924

## Description

### FIELD OF INVENTION

In mammals, the pH of the extracellular compartment, including interstitial fluids and blood, is strictly regulated and maintained at a constant value of 7.4. Acid sensing is a specific kind of chemoreception that plays a critical role in the detection of nociceptive pH imbalances occurring, for example, in conditions of cramps, trauma, inflammation or hypoxia (Lindahl, 1974). In mammals, a population of small-diameter primary sensory neurons in the dorsal root ganglia and trigeminal ganglia express specialized pH-sensitive surface receptors activated by increase of extracellular proton concentration (Bevan and Yeats, 1991). Acid sensitivity of sensory as well as central neurons is mediated by a family of proton-gated cation channels structurally related to *C. elegans* degenerins and mammalian epithelial sodium channels. This invention relates to these Acid Sensing Ion Channels (ASIC), particularly to a non-inactivating proton-gated channel, named hASIC3, its association with other channel subunits and uses thereof.

### BACKGROUND OF INVENTION

Tissue acidosis is associated with a number of painful physiological (e.g. cramps) and pathological conditions (e.g. inflammation, intermittent claudication, myocardial infarction). Experimentally, similar painful events can be reproduced by infusing low pH solutions into skin or muscle. Furthermore, the prolonged intradermal infusion of low pH solutions can mimic the characteristic hyperalgesia of chronic pain. To further characterize the effects of protons and their relation to pain, low pH solutions were applied to patch-clamped central and peripheral sensory neurons. Inward currents were induced when pH was dropped to acidic values, providing evidence for the existence of proton-activated ion channels. Several types of native currents were observed in sensory neurons from rat and human trigeminal and dorsal root ganglia:
■ rapidly inactivating currents;
■ non-inactivating currents; and
■ biphasic currents displaying a rapidly inactivating current followed by a non-inactivating current.

Other differences regarding ion selectivities were also reported. These results suggested the existence of several proton-gated ion channels. The prolonged pain induced by tissue acidification is most likely associated with a non-inactivating proton-gated ion channel.

### Cloned proton-gated ion channels

Three classes of mammalian proton-gated cation channels have recently been cloned in rat and named « ASIC » for Acid Sensing Ion Channels. Sequence analysis identifies them as members of the DEG/ENaC superfamily of ion channels. The putative membrane topology of ASIC receptors predicts two transmembrane spanning domains with both N- and C-termini in the intracellular compartment, as shown for the epithelial sodium channels (ENaC). The published ASIC receptors are described below:
1) **ASIC1A** (first reported as ASIC), displays a rapidly inactivating current with a pH₅₀ of 6.2 (Waldmann R. et al., *Nature* 1997; **386:** 173-7). N.B.: pH₅₀ indicates the pH at which peak inward current equals half the maximal value.
2) **ASIC1B** (initially reported as ASIC-β), is a splice variant of ASIC1A where the first 185 amino acids of ASIC1A are replaced by a distinct new sequence of 172 amino acids. ASIC1B shows similar current kinetics and pH₅₀ as those observed for ASIC1A (Chen C-C et al., Proc *Natl Acad Sci* 1998; **95:** 10240-5).
3) **ASIC2A** (first reported as MDEG, then MDEG1), displays a slowly inactivating current with a pH₅₀ of 4.05 (Waldmann R. et al., *J Biol Chem* 1996; **271:** 10433-6).
4) **ASIC2B** (first reported as MDEG2) is a splice variant of ASIC2A where the first 185 amino acids are replaced by a distinct new sequence of 236 amino acids (Lingueglia E. et al., *J Biol Chem* 1997; **272:** 29778-83). When expressed in heterologous expression systems, ASIC2B does not appear to be activated by protons.
5) **DRASIC,** which displays a biphasic current where the rapidly inactivating component has a pH₅₀ of 6.5 and the non-inactivating component a pH₅₀ of 3.5 (Waldmann R. et al., *J Biol Chem* 1997; **272:** 20975-8).

### Tissue distribution of cloned proton-gated ion channels

ASIC1A and ASIC2B mRNAs are present in both brain and sensory neurons. ASIC2A mRNA is detected in the central nervous system but is absent in sensory neurons. ASIC1B and DRASIC are exclusively expressed in sensory neurons, predominantly in small diameter neurons. The different ASIC subunits in brain (ASIC1A, ASIC2A, ASIC2B) as well as the ASICs in sensory neurons (ASIC1B, ASIC2B and DRASIC) appear to be coexpressed in the same neurons (Waldmann R. et al., *Curr Opinion Neurobiol* 1998; **8**: 418-24).

### Homo- and heteromultimeric assembly of proton-gated channel subunits

Ion channels are multimeric complexes, which result from the association of several identical (homopolymeric) and/or different (heteropolymeric) channel subunits. Except for ASIC2B, all ASIC subunits associate into homomultimeric complexes and yield the functional channels described above. In addition, certain ASIC subunits have been shown to form functional heteromultimeric channels with distinctive properties. Indeed, ASIC2B, which by itself does not give rise to a proton-gated channel, modifies the channel characteristics of ASIC2A and of DRASIC when coexpressed with either one or the other. The homomultimeric ASIC2A channel has a single exponential inactivation profile and is highly selective for sodium. When coexpressed with ASIC2B, the inactivation kinetics become biphasic with a slowly inactivating component that poorly discriminates between sodium (Na⁺) and potassium (K⁺) ions. Similarly, when DRASIC was coexpressed with ASIC2B, the sustained sodium-selective current of DRASIC became cation non-selective (pNa⁺=pK⁺) (Lingueglia E. et al., *J Biol Chem* 1997; **272**: 29778-83).

### Proton-gated channels are related to degenerins and mechanosensation

As mentioned above, ASICs belong to the DEG/ENaC superfamily of receptors, which also includes epithelial sodium channels (EnaC) involved in sodium homeostasis, the FMRF amide peptide-activated channel FaNaC from *Helix aspersa,* involved in neurotransmission, the ATP-gated cation channels, also involved in neurotransmission, as well as the degenerins of *Caenorhabditis elegans,* involved in mechanotransduction. As mentioned above, involvement of proton-gated ion channels in nociception seems likely. However, since many of the ASIC subunits are also expressed elsewhere than in sensory neurons, other functions than nocipeption must also be considered. In addition to their noxious effects, protons might play important roles as neurotransmitters as implied by reports of neuronal activity in response to fluctuations of pH. However, it still remains to be proven that sufficient pH changes can occur to activate ASIC channels with low pH₅₀, such as ASIC2A or ASIC2A+ASIC2B channels. Alternatively, ASIC channels might also be activated by other ligands, such as neuropeptides and neurotransmitters, or by mechanical energy, as suggested by their sequence homology with the other members of the DEG/EnaC superfamily.

### SUMMARY OF INVENTION

The object of the present invention is to provide the primary structure, functional characterization and tissue distribution of a human non-inactivating amiloride-sensitive proton-gated ion channel, designated herein as hASIC3.

As indicated above, Waldmann R. et al., *J Biol Chem* 1997; **272:** 29075-8 describes the cloning of rat DRASIC, a H⁺-gated cation channel, specified or sensory neurons that has both a rapidly inactivating component and a sustained component. However, as will become apparent from the comparison of the characteristics of hASIC3 and DRASIC, there are important differences between these two ion channels.

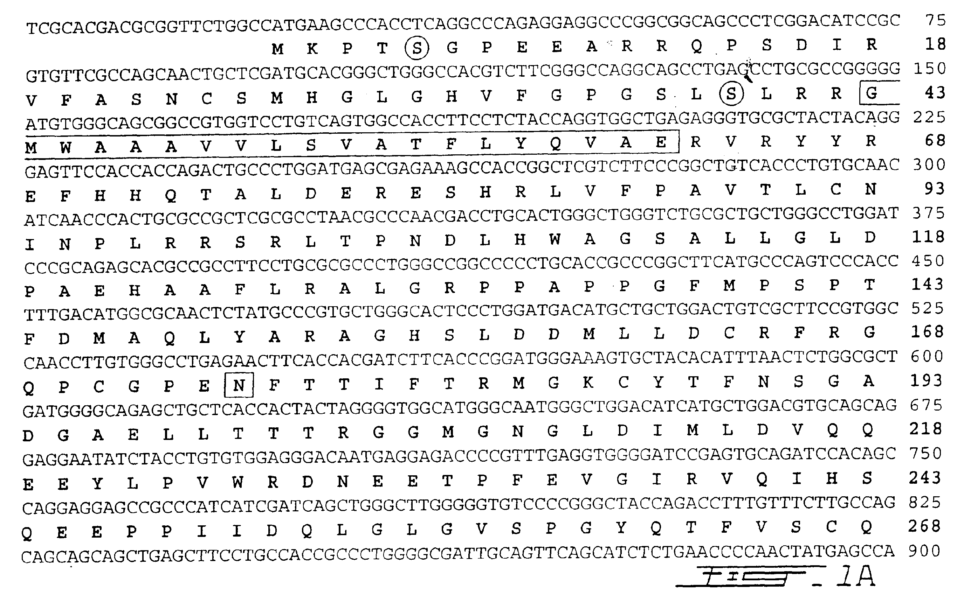

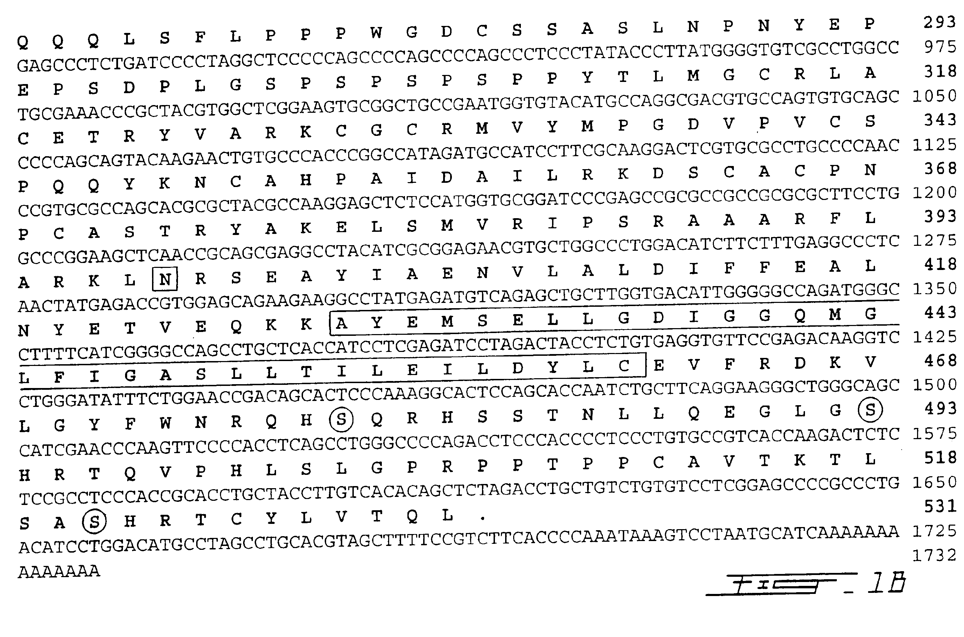

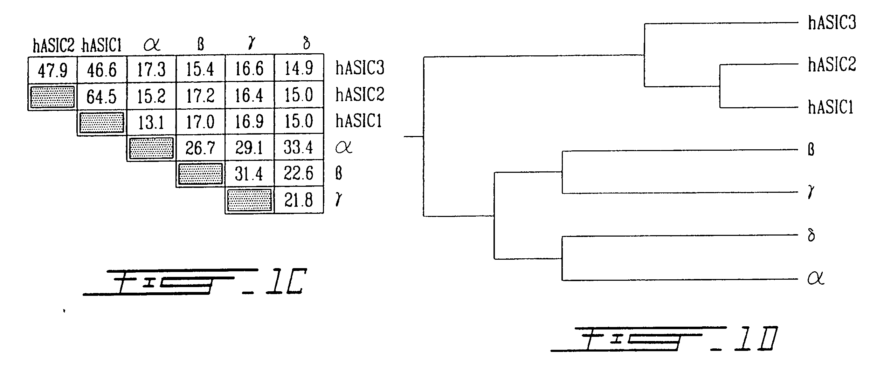

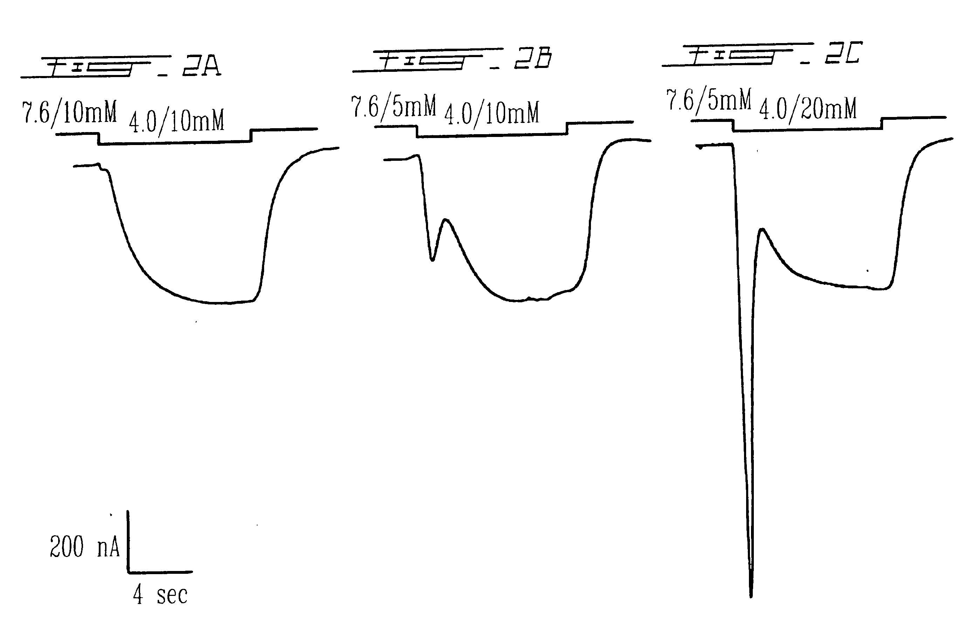

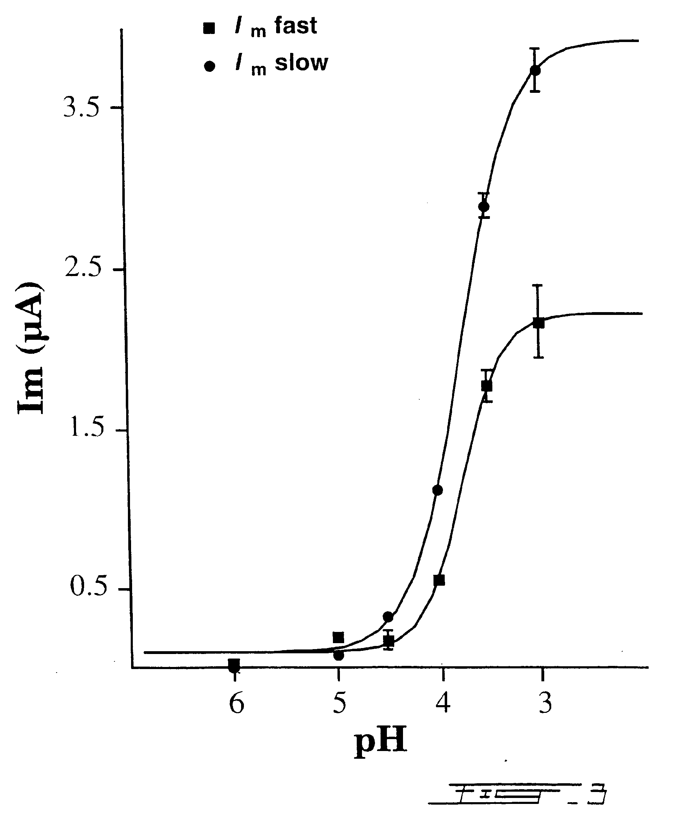

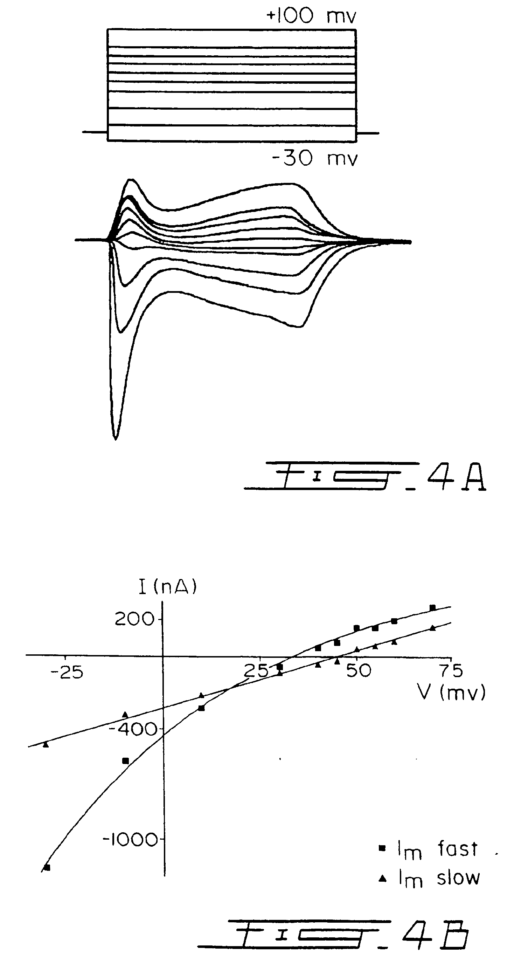

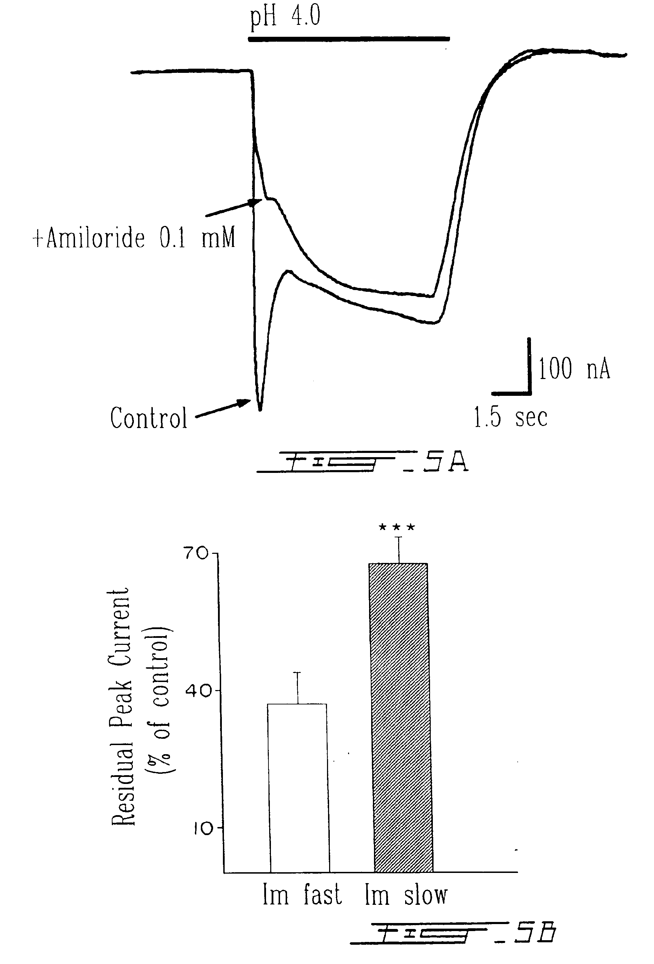

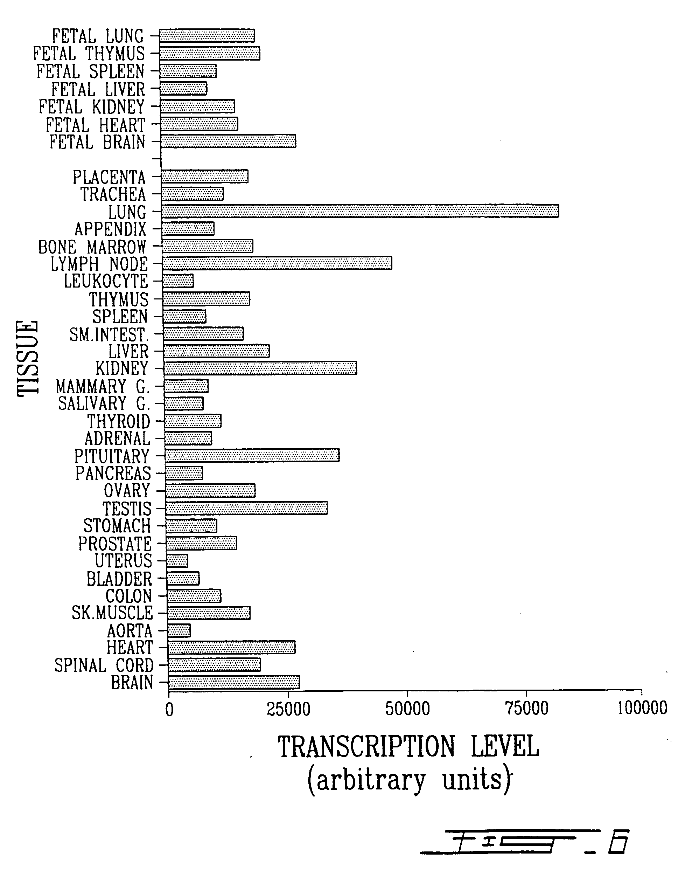

hASIC3 is not the orthologue of rat DRASIC as supported by the following evidence:
1) hASIC3 has 83% sequence identity with rat DRASIC while human and rat ASIC1A orthologues show an identity of 97.7% and human and rat ASIC2A orthologues show an identity of 99%. (Garcia-Anoveros J. et al., *Proc Natl Acad Sci* 1997; **94:** 1459-64).
2) Tissue distribution of hASIC3 is widespread throughout the body while rat DRASIC mRNA is restricted to sensory neurons (comparer Fig. 6 and Waldmann R. et al., *J Biol Chem* 1997; **272**: 20975-8)
3) hASIC3 and rat DRASIC biphasic currents display different properties. In the following discussion «fast component» will refer to the rapidly inactivating current and «slow component» will refer to the sustained current, which follows the fast component:
   ■ The fast and slow component of hASIC3 have similar pH₅₀s (3.66 and 3.82, respectively), while rat DRASIC has different pH₅₀s for the fast and slow components (6.5 versus 3.5, respectively) (compare Fig. 3 and Waldmann et al., *J Biol Chem* 1997; **272**: 29075-8).
   ■ The slow component of hASIC3 is inhibited by amiloride while the slow component of rat DRASIC is potentiated by amiloride (compare Fig. 5A and 5B and Waldmann et al., J Biol Chem 1997; **272**: 29075-8).
   ■ Reversal potentials for the fast and slow components of hASIC3 differed by 15mV (Eᵣₑᵥ fast= +33 mV and Eᵣₑᵥ slow = +48 mV), while both fast and slow components of rat DRASIC have the same Eᵣₑᵥ of +32 mV. (compare Fig. 4 and Waldmann et al., *J Biol Chem* 1997; 272: 20975-8).

Another aim of the present invention is to provide a DNA sequence encoding a novel subtype of a human non-inactivating amiloride-sensitive proton-gated ion channel, hASIC3 and derivatives thereof.

Accordingly, the present invention provides an isolated proton-gated cation channel, a corresponding isolated nucleic acid, methods for making the same, corresponding compositions/kits and recombinant host cells containing the same, and the use thereof in screening assays as set forth in the claims.

In one embodiment there is provided an isolated nucleic acid molecule, which consists essentially of the nucleotide sequence depicted in SEQ ID No. 1, and derivatives thereof.

In one embodiment, the isolated nucleic acid molecule of the present invention encodes a peptide consisting essentially of the amino acid sequence listed in SEQ ID No.: 2, and derivatives thereof.

In another embodiment the invention provides an isolated nucleic acid encoding a proton-gated cation channel subunit having the amino acid sequence of SEQ ID NO:2 or a variant of that sequence having at least 531 amino acids and 85% identify therewith, wherein the proton-gated cation channel displays a biphasic current and the slow component of the biphasic current is amiloride-sensitive.

RNA encoding the hASIC3 receptor, transcribable from DNA in accordance with the present invention (SEQ ID No.: 1) and substantially free from other RNAs, also forms part of the invention, and may be useful for a number of purposes including hybridization sturies, *in vitro* translation as well as translation in appropriate *in vivo* systems such as *Xenopus* oocytes.

The present invention also relates to complete and/or partial complementary and/or antisense nucleotide sequences corresponding to the nucleotide sequence listed in SEQ ID No. 1.

In accordance with the present invention, there is provided a vector, preferably an expression vector, selected from the group consisting of plasmid, phage, retrovirus, baculovirus, adenovirus and integration elements, which include the isolated nucleic acid molecule of the present invention.

The present invention also relates to host cells transformed or transfected with a vector as described above. Host cells may be prokaryotic or eukaryotic and include mammalian cells (such as COS, CHO cells and human embryonic kidney cells, HEK293), insect cells, yeast (such as *Saccharomyces cerevisiae*) and bacteria such as *Escherichia coli*). Host cells will either transiently express hASIC3 and/or derivatives thereof, as in the case of COS cells, or be stably transfected with a vector carrying the hASIC3 sequence and/or derivatives thereof. A CHO cell line or any other cell line that stably expresses hASIC3 and/or any derivatives thereof can be used for electrophysiological, calcium-influx, ion-imaging, ligand-binding, affinity purification, immunoprecipitation, western blotting and immunoblotting studies. Host cells which do not express the receptor may still be useful as cloning hosts.

Another object of the present invention is to provide a method to isolate a ligand that will bind the hASIC3 protein, or any derivative thereof.

A hASIC3 and/or a derivative thereof prepared by recombinant DNA technology in accordance with the invention has a number of uses, either *in situ* in the membrane of the expression host or in *in vitro* systems. In particular, the receptor can be used as a screen for ligands and/or compounds useful in a variety of human (or other animal) diseases and conditions, such as pain, inflammation, ischemia and neurodegenerative disorders. Ligands refers to any chemical or biological entity that binds to any intracellular and/or extracellular region or portion of the hASIC3 and/or its derivatives. Such ligands include compounds present in combinatorial chemical libraries, peptide phage display libraries, extracts containing unknown compounds (for example plant extracts, marine life extracts, toxins, venoms), as well as biological molecules such as polyclonal and/or monoclonal antibodies, neurotransmitters, peptides or inorganic ions and metals.

Also provided is the use of a recombinant host cell according to the invention for screening compounds useful as proton-gated cation channel ligands. A nucleic acid encoding a channel of the invention can also be used for screening compounds useful as proton-gated cation channel ligands.

The invention also provides compositions or kits for screening compounds useful as proton-gated channel ligands, which comprise at least one of a channel according to the invention, a nucleic acid according to the invention, a recombinant vector according to the invention, a recombinant host cell according to the invention, and any suitable auxiliary component.

For example, in accordance with the present invention there is provided a method of using the isolated nucleic acid molecule Isited in SEQ ID No. 1, or a sequence which hybridizes under stringent conditions to the sequence listed in SEQ ID No. 1, to produce a peptide consisting essentially of the amino acid sequence listed in SEQ ID No. 2, which comprises the steps of:
a) transforming a host with a DNA sequence capable of encoding the peptide
b) incubating the host under conditions which allow the protein sequence to be expressed and
c) isolating by all means the protein from the host.

Also provided is a method of producing a proton-gated cation channel according to the invention comprising incubating a recombinant host cell of the invention under conditions which allow the nucleic acid sequence to be expressed, and isolating the protein from the recombinant host cell.

Recording or imaging the activity of the protein from the host can be performed to confirm or monitor the activity of the protein. This method can be reiterated, this time, with two or more DNA sequences encoding two or more different proteins, which results in the obtention of heteropolymeric channels.

Derivatives of hASIC3 include functional and/or structural variants as described below:
■ Derivatives of hASIC3 include molecules whose sequence differs from the hASIC3 sequence by a modification and/or substitution and/or insertion and/or deletion of one or several amino acid residues as long as this modification and/or substitution and/or insertion and/or deletion does not modify the functional and/or structural properties of the hASIC3 channel, mainly the activation by protons. The amino acid sequence of derivatives must be at least 85% or greater identical to the amino acid sequence of hASIC3. Such derivatives can be synthetized and/or analysed by a person skilled in the art following established techniques.
■ Derivatives of hASIC3 also include molecules whose sequence differs from the hASIC3 sequence by a modification and/or substitution and/or insertion and/or deletion of one or several amino acid residues even if this modification and/or substitution and/or insertion and/or deletion does modify the functional and/or structural properties of the hASIC3 channel, rendering it non-or differently responsive to protons.

Examples of derivatives are hASIC3-like channel subunits corresponding to the partial nucleotide sequences (Expressed Sequence Tag available from public dbest databases, such as EST # A1024055, AA628357, AA448259, AA449322, hASIC3 channel subunits epitope-tagged on N-terminus and/or C-terminus, as well as hASIC3 channel subunit where the first 150-200 amino acids were subtituted by a new and different amino acid sequence, in a similar fashion as reported for ASIC1A and ASIC1 B, and ASIC2A and ASIC2B.

The present invention relates to the association of hASIC3 and derivatives thereof with other channel subunits such as the proton-gaged ion channel subunits and derivatives thereof (for example ASIC1A, ASIC1B, ASIC2A, ASIC2B), or other channel subunits related to the DEG/EnaC superfamily of receptors, such as alpha-, beta-, gamma-, delta-EnaC subunits or the P2x ATP-gated ion channel subunits. Such associations include interactions between subunits of different species, but the preferred association are between subunits from the same species.

An example of heteromeric association between hASIC3 and rat P2x2 is given herein below. The resulting new channel is a novel proton-gated ion channel, which displays higher sensitivity to pH. Indeed, when hASIC3 and P2x2 are coinjected into *Xenopus* oocytes, inward currents can be activated with slight acidification to pH 6.5, while the homomeric hASIC3 requires a pH of 4.0. This different pharmacological property proves that hASIC3 and P2x2 physically associated to generate a new ion channel.

Also provided is an isolated human proton-gated cation channel comprising a subunit that comprises an amino acid sequence that is at least 85% identical to the amino acid sequence of SEQ ID NO:2, wherein the proton-gated cation channel displays a biphasic current when activated by an extracellular proton concentration which is below physiological pH, and wherein the slow component of the biphasic current is amiloride-sensitive.

The invention further provides an isolated proton-gated cation channel comprising a subunit having the amino acid sequence of SEQ ID NO:2 or a variant of that sequence having at least 531 amino acids and at least 85% identity therewith, wherein the channel displays a biphasic current when activated by an extracellular proton concentration which is below physiological pH and the slow component of the biphasic current is amiloride-sensitive.

### DESCRIPTION OF INVENTION

This invention will be described by way of specific embodiments, examples and figures, which purpose is to illustrate the invention rather than to limit its scope.

### BRIEF DESCRIPTION OF THE DRAWINGS

**Figure 1.** Nucleotide and predicted amino acid sequence of hASIC3 cDNA (SEQ ID No. 1 and No. 2, respectively) (A), deposited in Genbank database under the accession number AF057711. Two stretches of 20-34 amino acids corresponding to potential transmembrane domains, identified in Kyte-Doolitle hydrophobicity plot, are highlighted. Consensus phosphorylation sites in intracellular domains and N-glycosylation sites in extracellular domain are indicated by circled and boxed residues, respectively. Homology (in %) of hASIC3 (B) and phylogenetic relationships (C) with known members of the human ASIC/ENaC family. Protein dendrogram generated using UPGMA algorithm (Geneworks 2.5.1, Oxford Molecular Group).
**Figure 2.** Biphasic current phenotype of homomeric hASIC3 channels: effects of increasing rates of pH change. Oocytes were clamped at -70 mV and continuously perfused with Ringer's buffer containing 10 mM HEPES at pH 7.6. pH was then dropped to 4.0 for 10 sec with increasing pH gradients obtained by raising the buffer capacity differential between control and test buffers: A. pH 7.6 to pH 4.0 in 10 mM HEPES; B. pH 7.6 in 5 mM to pH 4.0 in 10 nM HEPES; and C. pH 7.6 in 5 mM to pH 4.0 in 20 mM HEPES. Controls done with the test buffers at pH 7.6 did not activate the hASIC3 channel. Oocytes injected with injection buffer alone showed no inward currents. The amplitude of the early but not the late component, and thus the ratio of early/late peak currents, appears to be very sensitive to the speed at which the pH drops from normal to acid pH values.
**Figure 3.** Dose-response curves of pH activation of hASIC3 currents. Dose-response curves were constructed in 20 mM HEPES buffered at different pH values from 6.0 to 3.0. Peak currents of fast and slow currents were analysed with the four parameter logistic equation and the partial F test for statistical comparison. Each point represents mean +/- SEM from this typical experiment. Apart from the maximal response, no other significant difference was observed between both curves.
**Figure 4.** Current-voltage (I/V) relationship of the fast and slow hASIC3 currents. Recordings were done in Ringer's buffer containing (in mM): NaCl 115, KCI 2.5, CaCl₂ 1.8 and HEPES 5, pH 7.6. The I/V relationship was established by measuring peak currents of both fast and stow responses to pH 4.0 (applied 1 sec after voltage step) at different membrane potentials, after substracting background currents recorded without pH applications (A). Peak current values were plotted (B) and reversal potential estimated from linear (slow current) and nonlinear (fast current) regression analysis. Panels A and B represent a typical experiment where reversal potentials were 33.4 mv and 44.8 mv respectively for the fast and slow currents.
**Figure 5.** Differential sensitivity of the fast and slow hASIC3 currents to amiloride. hASIC3 currents were activated by pH 4.0 in the presence and absence of 100 µM amiloride (A). Inhibition by amiloride was much stronger on the fast than the sustained current. Data in B represent mean +/- SEM (n= 17) of residual peak currents during amiloride application for the fast (37.2 +/- 6.4%) and sustained (72.0 +/- 3.8%) components expressed as percent of controls. Statistical significance (P) was evaluated by unpaired two-tailed t-test (*** P<0.001).
**Figure 6.** Distribution of hASIC3 mRNA in normal human tissues. High stringency hybridization of radiolabeled hASIC3 cDNA on human polyA+ RNA isolated from brain, spinal cord, internal tissues as well as from 17-28 weeks fetal.tissues was quantitated by densitometric analysis in phosphorimaging. Amounts of polyA+ RNA target were normalized across tissues to allow direct comparisons of transcription levels (see Materials and Methods for details).

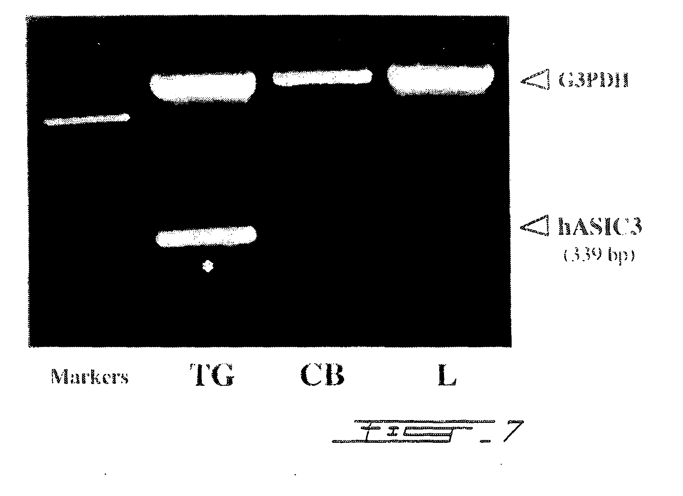

**Figure 7.** High levels of transcription of hASIC3 gene in sensory ganglia enriched in nociceptive neurons. Localization of hASIC3 and reporter G3PDH mRNAs in human trigeminal ganglia (TG), cerebellum (CB) and lung (L) using RT-PCR amplification with specific exact match primers (see Materials and Methods section for details).

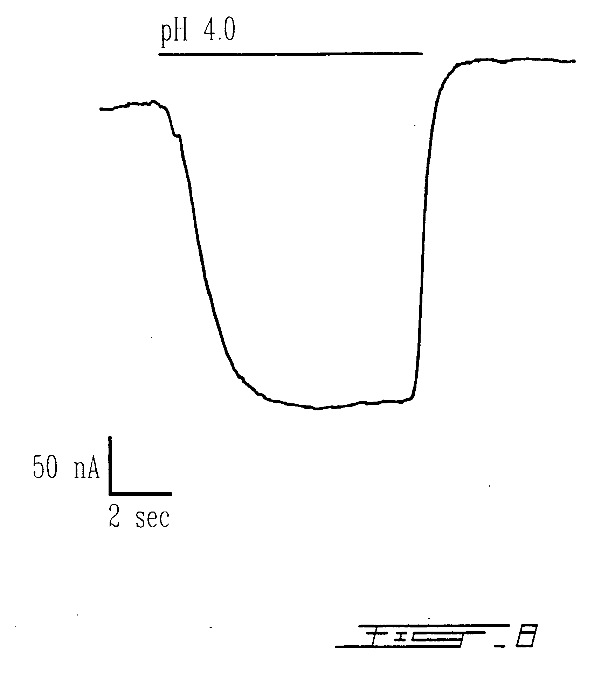

Figure 8. Illustration of a recording of non-inactivating cation current induced by strong acid (pH 4.0) in *Xenopus* oocytes injected with hASIC3 clone alone in pcDNA3 vector.

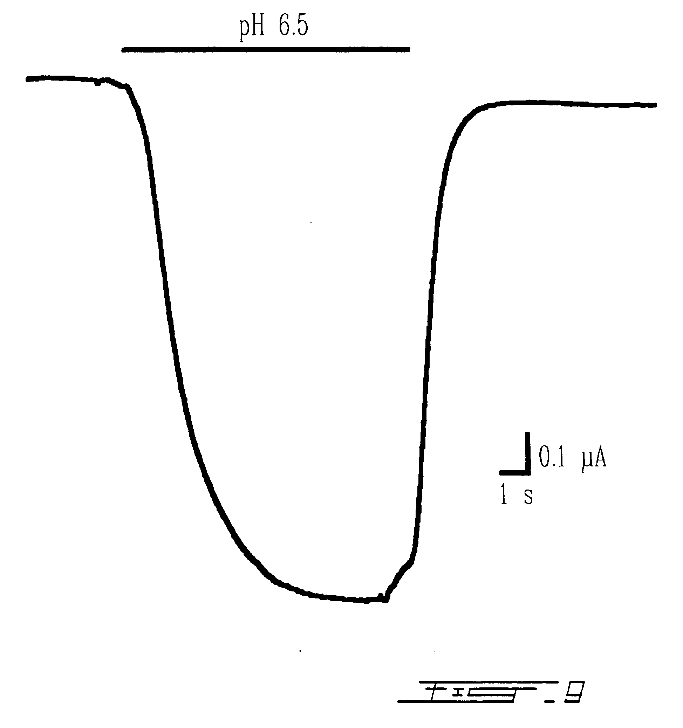

Figure 9. Illustration of a recording of non-inactivating cation current induced by weak acid (pH 6.5) in *Xenopus* oocytes coinjected with hASIC3 clone and rat P2x2 clone, both in pcDNA3 vector.

### EXAMPLE 1: Characterization of a human non-inactivating proton-gated ion channel, hASIC3

### Introduction

Acid sensing is a specific kind of chemoreception that plays a critical role in the detection of nociceptive pH imbalances occurring in conditions of cramps, trauma, inflammation and hypoxia (Lindahl, 1974). In mammals, a population of small-diameter primary sensory neurons in the dorsal root ganglia and trigeminal ganglia express specialized pH-sensitive surface receptors activated by increase of extracellular proton concentration (Bevan and Yeats, 1991). Native electrophysiological responses of sensory neurons to applications of pH 5.8-6.5 are characterized by a fast desensitizing inward current followed by a slow sustained current (Krishtal and Pidoplichko, 1981). Elucidating the native molecular composition of proton sensors in human sensory neurons will be an important step in the rational development of a novel class of analgesics. A family of genes coding for neuronal proton-gated channels subunits has been recently discovered (Garcia-Anoveros et al., 1997; Waldmann et al., 1997). Heterologously expressed amiloride-sensitive homomeric rat ASIC (Acid Sensing Ion Channel) (Waldmann et al., 1997) responds to small pH changes by a fast desensitizing sodium-selective current, while MDEG1 (Mammalian DEGenerin 1) (Waldmann et al., 1996) and DRASIC (Dorsal Root ganglia ASIC) (Waldmann et al., 1997) require drastic pH changes to gate desensitizing and biphasic currents, respectively. ASIC and MDEG1 can associate together to generate a heteromeric channel activated at low pH (<5) with unique kinetics and ionic selectivities (Bassilana et al., 1997). A neuronal splicing variant of MDEG1 was shown to modulate DRASIC biophysical properties by heteromeric association (Lingueglia et al., 1997). These proton-gated channels share a putative two-transmembrane domain topology and co-localization in small diameter capsaicin-sensitive sensory neurons with P2X ATP-gated channels (North, 1997). From their sequence, they belong to an expanding gene superfamily including mammalian epithelial sodium channels (Canessa et al., 1994), pickpocket (PPK) and ripped pocket (RPK) subunits from *Drosophila* (Adams et al., 1998), degenerins of *C. elegans* (Corey and Garcia-Anoveros, 1996), and the FMRFamide-gated channel of *Helix aspersa* (Lingueglia et al., 1995). Despite their potential importance in monitoring pH changes in central nervous system and sensory pathways, human proton receptor genes have not yet been functionally characterized. We report here for the first time the heterologous expression of a human proton-gated channel, as well as significant inter-species differences observed both in functional properties and regional distributions of acid sensors.

### Material and Methods

### Molecular cloning

Using the tblastn algorithm, virtual screening of the dbEST database of NCBI (Lennon et al., 1996) with probes corresponding to the protein motif LXFPAVTLCNXNXXRXS, conserved in all known members of the degenerin/ENaC/ASIC family, led to the identification of human EST sequences encoding a novel member of the proton sensor gene family (Genbank accession numbers AA449579 and AA429417). The clone tagged by 5' EST AA449579 and by 3' EST AA449322 from a total fetus cDNA library was sequenced on both strands using walking primers and an ALF DNA sequencer (Pharmacia-LKB). Full-length hASlC3 was directionally subcloned into unique EcoRl and Notl sites of eukaryotic vector pcDNA3 (Invitrogen) for CMV promoter-driven heterologous expression in *Xenopus* oocytes.

### Electrophysiology in Xenopus oocytes

Oocytes surgically removed from adult *Xenopus* laevis were treated for 2 h at room temperature with type II collagenase (Gibco-BRL) in Barth's solution under constant agitation. Selected oocytes at stage IV-V were defolliculated manually before nuclear micro-injections (Bertrand et al., 1991) of 5 ng of hASlC3 in pcDNA3 vector. After 2-4 days of expression at 19⁻C in Barth's solution containing 50Êg/ml gentamycin, currents were recorded in the two-electrode voltage clamp configuration using an OC-725B amplifier (Warner Instruments). Whole cell currents were acquired and digitized at 500 Hz on a Macintosh IIci computer with an A/D NB-MIO16XL interface (National Instruments) then recorded traces were post-filtered at 100 Hz in Axograph (Axon Instruments). Agonist, amiloride and wash solutions were prepared in a modified Ringer's solution containing 115 mM NaCl, 2.5 mM KCI, 1.8 mM CaCl₂ in 5-20 mM HEPES (Sigma) buffer adjusted with NaOH or HCl at pH 2 to 8 and applied on oocytes by constant perfusion (10-12 ml/min) at room temperature. Mean values ± SEM corresponded to measurements from a minimum of 5 oocytes.

### RT-PCR and mRNA dot blot hybridization

Total RNA from post-mortem samples of normal human trigeminal ganglia were isolated using Trizol reagent (Gibco-BRL), then 1 Eg was subjected to random-primed reverse transcription using Superscript (Gibco-BRL). Around 100 ng of RT-cDNA was used as template for PCR with Expand DNA polymerase (Boerhinger-Mannheim). Specific hASIC3 primers TCAGTGGCCACCTTCCTCTA (forward) and ACAGTCCAGCAGCATGTCATC (reverse) were used to amplify the region corresponding to nucleotides 175-513 (Fig.IA). After initial template denaturation of 2 min at 94-C, thermal cycles consisted of 45 sec at 94⁻C, 45 sec at 55⁻C and 2 min at 72⁻C for 30 cycles. Molecular identity and homogeneity of PCR products were checked by sizing and specific restriction patterns. Initial sample loading was checked by co-amplification of glyceraldehyde 3-phosphate dehydrogenase (G3PDH) housekeeping mRNA. RNA samples not subjected to reverse transcription but PCR amplified in identical conditions provided our negative controls.

Known amounts of human polyA+ RNA (89-514 ng), isolated from various fetal and adult normal tissues and normalized for the transcription levels of several housekeeping genes (Clontech), were dot-blotted and probed with the [32P]-labeled EcoRI-XbaI fragment of hASlC3 cDNA at high stringency (final elution at 65⁻C in 0.3 X SSC buffer for 10 min). After exposure for 16 hours, hybridization signals were acquired and quantitated using a Storm phosphorimager (Molecular Dynamics), then analyzed in densitometry with ImageQuant software (Molecular Dynamics).

### Results

### Primary structure of hASIC3 channel subunit

Sequence analysis of the 1.7 kb-long hASIC3 polyA+ mRNA revealed an open reading frame encoding 531 amino acids (Fig. IA), with initiation of translation at the proximal Met codon located at position nt. 22. The predicted molecular weight of 59 kDa for the immature protein was confirmed by *in vitro* translation (data not shown). According to the current topological model based on primary structure analysis and biochemical tests, a large domain of 365 amino acids faces the extracellular side of the plasma membrane (Canessa et al., 1994). In this extracellular domain, a total of 15 cysteine residues are highly conserved in the ASIC family, with the exception of Cys267 being absent in human BNaC1 (hASIC2) only. Two potential sites for Asn-linked glycosylation, Asn175 and Asn398, are located in this Cysteine-rich loop. Consensus sites for phosphorylation by casein kinase II (Ser5) and by protein kinase C (Ser39, Ser478, Ser493, Ser521) are found in the intracellular N-terminal domain of hASlC3 as well as in the intracellular C-terminal domain (Fig. IA). The hASlC3 subunit displays 83% of identity with rat DRASIC subunit at the amino acid level, 48% with human BNaC2 (hASIC1) and 47% with BNaC1 (hASlC2) (Fig. 1B). Therefore hASIC3 belongs to the proton-gated channel family, itself a branch of the degenerin/ENaC/FMRFamide-gated channel phylogenetic tree (Fig. 1C).

### Functional and pharmacological properties of homomeric hASlC3 channels

When heterogously expressed in *Xenopus* oocytes, hASlC3 subunits assemble into functional homomeric channels activated by low extracellular pH (Fig. 2A). Rapid changes of extracellular pH (Fig. 2B-C) revealed a biphasic response. This unique phenotype was characterized by a fast and rapidly desensitizing current followed by a slow and sustained current which returned to baseline only upon return to physiological pH. The relative amplitude of the fast current appeared dependent on the slope of the pH gradient applied (Fig. 2A-C). However, we found the pH sensitivity of the two hASIC3-mediated currents to be almost identical with a pH50 of 3.66 +/- 0.06 (fast) vs 3.82 +/- 0.04 (slow) (Fig. 3). The positive cooperativity reflected in the dose-response curve profile, nHfast=1 57 +/- 0.3 and nHslow=1 55 +/- 0. 17, indicated that at least two protonations on two subunits are required in order to gate the cation channel. To study possible differences of ionic selectivity between fast and slow components, we performed a current-voltage relationship at pH 4.0 in normal Ringer. The peak amplitude of the fast component displayed some voltage-dependence from its slight inward rectification, while the slow and sustained component was ohmic in the range from -70 to + 70 mv (Fig. 4B). Furthermore, although both reversal potentials were greater than 30 mv as expected for channels conducting mainly sodium, we measured a _Erev= +15 +/- 3.2 mv (p < 0.01) between the fast (+32.9 +/- 4.4 mv) and the slow component (+48.2 +/- 4.8 mv) (Fig. 4A-B). These two phases of proton-induced hASlC3 current differed also by their sensitivity to the antagonist amiloride. Co-application of 100 micro M amiloride with pH 4.0 in conditions of biphasic response demonstrated a more efficient blockade of the fast (62.8 +/- 6.5%) than of the slow current (28.7 +/- 4.6%) by amiloride (Fig. 5AB).

### Central and peripheral distribution of hASIC3 gene expression

As a rough index of anatomical distribution and mRNA abundance, we noticed several cDNAs encoding hASlC3 in total fetus and testis cDNA libraries representated in the dbEST database. Results obtained in RNA hybridization at high stringency confirmed that the hASIC3 gene is transcribed in a wide spectrum of internal organs as well as in the central nervous system (Fig. 6). In the adult stage, hASlC3 transcripts were detected in lung, lymph nodes, kidney, pituitary, heart and testis as well as in brain and spinal cord. A developmental up-regulation of hASlC3 gene expression was apparent when comparing fetal vs adult mRNA levels in lung and kidney (Fig. 6). Thus hASlC3 subunit expression is not restricted exclusively to sensory ganglia as is rat DRASIC, explaining our decision to use a chronological nomenclature that is distribution-independent. The potentially important function of non-inactivating proton-gated channels in nociceptive sensory neurons was nevertheless confirmed by the detection of high levels of ASIC3 mRNA in adult human trigeminal ganglia using RT-PCR (Fig. 7).

### EXAMPLE 2: Heteromultimeric channel composed of hASIC3 and rat P2x2

To demonstrate the possibility of heteromultimeric association between hASIC3 and/or derivatives thereof and other ion channel subunits, we have coexpressed the hASIC3 and rat P2x2 constructs in *Xenopus* oocytes to verify if the presence of an ATP-gated ion channel subunit can modify hASIC3 channel properties. P2X2 displays important similarities with hASIC3, such as overall membrane topology (two transmembrane spanning domains, cysteine rich extracellular domain), sensitivity to pH (response to ATP of the P2X2 homomultimeric channel is strongly potentiated by acid pH), and tissue distribution (both hASIC3 and P2X2 are colocalized in sensory neurons).

There is shown in Fig. 8 the recording of non-inactivating cationic current induced by strong acid (pH 4.0) in *Xenopus* oocytes injected with the hASIC3 clone alone in pcDNA3 vector. These data demonstrate that hASIC3 alone can associate in functional homomeric cation channels.

There is shown in Fig. 9 the recording of non-inactivating cation current induced by weak acid (pH 6.5) in *Xenopus* oocytes coinjected with hASIC3 clone and rat P2x2 clone, both in pcDNA3 vector. These data demonstrate that the co-expression of hASIC3 and rat P2x2 changes the pH sensitivity of homomeric hASIC3 or leads to the formation of heteromultimeric pH-sensitive channels.

### References

Adams C.M., Anderson M.G., Motto D.G., Price M.P., Johnson W.A., and Welsh M.J. (1998) Ripped pocket and pickpocket, novel Drosophila DEG/ENaC subunits expressed in early development and in mechanosensory neurons. J. Cell Biol. 140, 143-152.
Bassilana F., Champigny G., Waldmann R., de Weille J.R., Heurteaux C., and Lazdunski M. (1997) The acid-sensitive ionic channel subunit ASIC and the mammalian degenerin MDEG form a heteromultimeric H+-gated Na+ channel with novel properties. J. Biol. Chem. 272, 28819-28822.
Bertrand D., Cooper E., Valera S., Rungger D., and Ballivet M. (1991) Electrophysiology of neuronal nicotinic acetylcholine receptors expressed in Xenopus oocytes following nuclear injection of genes or cDNAs. In: Methods in Neurosciences (Conn M.P., ed.) pp 174-193. New York: Academic.
Bevan S ., and Winter J. (1995) Nerve growth factor (NGF) differentially regulates the chemosensitivity of adult rat cultured sensory neurons. J. Neurosci. 15, 4918-4926.
Bevan S., and Yeats J. (1991) Protons activate a cation conductance in a sub-population of rat dorsal root ganglion neurones. J. Physiol. 433, 145-161.
Canessa C.M., Merillat A.M., and Rossier B.C. (1994) Membrane topology of the epithelial sodium channel in intact cells. Am. J. Physiol. 267, C1682-1690,
Canessa C.M., Schild L., Buell G., Thorens B., Gautschi I., Horisberger J.-D., and Rossier B.C. (1994) Amiloride-sensitive epithelial Na+ channel is made of three homologous subunits. Nature 367, 463-467.
Corey D.P., and Garcia-Anoveros J. (1996) Mechanosensation and the DEG/ENaC ion channels. Science 273, 323-324.
Coscoy S., Lingueglia E., Lædunski M., and Barbry P. (1998) The Phe-Met-Arg-Phe-amide-activated sodium channel is a tetramer. J. Biol. Chem. 273, 8317-8322.
De LÇan A., Munson P.J., and Rodbard D. (1978) Simultaneous analysis of families of sigmoidal curves: application to bioassay, radioligand assay, and physiological dose-response curves. Am. J. Physiol. 235, E97-E102.
Dray A., and Perkins M. (1993) Bradykinin and inflammatory pain. Trends Neurosci. 16, 99-104.
Firsov D., Gautschi I., Merillat A.-M., Rossier B.C., and Schild L. (1998) The heterotetrameric architecture of the epithelial sodium channel (ENaC). The EMBO J. 17, 344-352.
Garcia-Anoveros J., Derfler B., Neville-Golden J., Hyman B.T., and Corey D.P. (1997) BNaCl and BNaC2 constitute a new family of human neuronal sodium channels related to degenerins and epithelial sodium channels. Proc. Natl. Acad. Sci. USA 94, 1459-1464.
Ishibashi K., and Marumo F. (1998) Molecular cloning of a DEG/ENaC sodium channel from human testis. Biochem. Biophys. Res. Comm. 245, 589-593.
Kleyman T.R., and Cragoe E.J. (1988) Amiloride and its analogs as tools in the study of ion transport. J. Membr. Biol. 105, 1-21.
Konnerth A., Lux H.D., and Morad M. (1987) Proton-induced transformation of calcium channel in chick dorsal root ganglion cells. J. Physiol. 386, 603-33.
Krishtal O.A., and Pidoplichko V.I. (1981) A receptor for protons in the membrane of sensory neurons may participate in nociception. Neuroscience 6, 2599-2601.
Krishtal O.A., Osipchuk Y.V., Shelest T.N., and Smirnoff S.V. (1987) Rapid extracellular pH transients related to synaptic transmission in rat hippocampal slices. Brain Res. 436, 352356.
Lennon G., Auffray C., Polymeropoulos M., and Soares M.B. (1996) The I.M.A.G.E. consortium; an integrated molecular analysis of genomes and their expression. Genomics 33, 151-152.
Lindahl O. (1974) Pain- a general chemical explanation. Advances Neurol. 4, 45-47.
Lingueglia E., Champigny G., Lazdunski M., and Barbry P. (1995) Cloning of the amiloride-sensitive FNIRFamide peptide-gated sodium channel. Nature 378, 730-733.
Lingueglia E., de Weille J.R., Bassilana F., Heurteaux C., Sakai H., Waldmann R., and Lazdunski M. (1997) A modulatory subunit of acid sensing ion channels in brain and dorsal root ganglion cells. J. Biol. Chem. 272, 29778-29783.
North R.A. (1997) Families of ion channels with two hydrophobic segments. Current Opinion in Cell Biology 8, 474-483.
Price M.P., Snyder P.M., and Welsh M.J. (1996) Cloning and expression of a novel human brain Na+ channel. J. Biol. Chem. 271, 7879-7882.
Snyder P.M., Cheng C., Prince L.S., Rogers J.C., and Welsh MJ. (1998) Electrophysiological and biochemical evidence that DEG/ENaC cation channels are composed of nine subunits. J. Biol. Chem. 273, 681-4.
Tavernarakis N., Shreffler W., Wang S., and Driscoll M. (1997) unc-8, a DEG/ENaC family member, encodes a subunit of a candidate mechanically gated channel that modulates C. elegans locomotion. Neuron 18, 107-119.
Waldmann R., Bassilana F., de Weille J., Champigny G., Heurteaux C., and Lazdunski, M. (1997) Molecular cloning of a non-inactivating proton-gated Na+ channel specific for sensory neurons. J. Biol. Chem. 272, 20975-20978.
Waldmann R., Champigny G., Bassilana F., Heurteaux C., and Lazdunski M. (1997) A proton-gated cation channel involved in acid-sensing. Nature 386, 173-177.
Waldmann R., Champigny G., Voilley N., Lauritzen I., and Lazdunski M. (1996) The mammalian degenerin MDEG, an amiloride-sensitive cation channel activated by mutations causing neurodegeneration in Caenorhabditis elegans. J. Biol. Chem. 271, 0433-10436.

## Claims

1. An isolated proton-gated cation channel comprising a subunit having the amino acid sequence shown in Figure 1A/B or a variant of that sequence having at least 531 amino acids and at least 85% identity therewith, wherein the proton-gated cation channel displays a biphasic current and the slow component of the biphasic current is inhibited by amiloride.

2. A channel according to Claim 1, which is a homopolymeric channel.

3. A channel according to Claim 1, which is a heteropolymeric channel.

4. A channel according to Claim 3 having at least one subunit which belongs to the degenerin/ENaC channel superfamily.

5. A channel according to Claim 3 having at least one subunit which belongs to the P2X ATP-gated channel family.

6. A channel according to Claim 5 wherein the P2X family subunit is P2X2.

7. Use of a channel according to any of Claims 1 to 6 for screening compounds useful as proton-gated cation channel ligands.

8. An isolated nucleic acid encoding a proton-gated cation channel subunit having the amino acid sequence of shown in Figure 1A/B or a variant of that sequence having at least 531 amino acids and at least 85% identity therewith, wherein the proton-gated cation channel displays a biphasic current and the slow component of the biphasic current is inhibited by amiloride.

9. A nucleic acid encoding a proton-gated cation channel subunit as defined in Claim 1 and another cation channel subunit as defined in any one of Claims 4 to 6.

10. A recombinant vector comprising a nucleic acid as defined in Claim 8 or 9.

11. A method of producing a proton-gated cation channel according to any of Claims 1 to 6 comprising expressing a nucleic acid sequence according to Claim 8 or 9, wherein said method is not a method for treatment of the human or animal body by surgery or therapy or a diagnostic method practised on the human or animal body.

12. A recombinant host cell comprising a vector according to Claim 10 which is capable of expressing the cation channel according to Claim 1 or 2, wherein said host cell is not present in the human body.

13. A recombinant host cell comprising a vector according to Claim 10 which is capable of expressing the cation channel according to any of Claims 3 to 6, wherein said host cell is not present in the human body.

14. A method of producing a proton-gated cation channel according to any of Claims 1 to 6 comprising incubating the recombinant host cell of Claim 12 or 13 under conditions which allow the nucleic acid sequence to be expressed, and isolating the protein from the recombinant host cell.

15. Use of a recombinant host cell according to Claim 13 or 14 for screening compounds useful as proton-gated cation channel ligands.

16. Use of a nucleic acid encoding a channel according to any of Claims 1 to 6 for screening compounds useful as proton-gated cation channel ligands.

17. A composition or kit for screening compounds useful as proton-gated cation channel ligands, which comprises at least one of a channel according to any of Claims 1 to 6, a nucleic acid according to Claim 8 or 9, a recombinant vector according to Claim 10, a recombinant host cell according to Claim 12 or 13, and any suitable auxiliary component.

18. A composition or kit according to Claim 17 wherein the ligands are analgesics.

19. An isolated human proton-gated cation channel comprising a subunit that comprises an amino acid sequence that is at least 85% identical to the amino acid sequence shown in Figure 1A/B wherein the proton-gated cation channel displays a biphasic current when activated by an extracellular proton concentration which is below physiological pH, and wherein the slow component of the biphasic current is inhibited by amiloride.

20. An isolated proton-gated cation channel subunit consisting of the amino acid sequence of shown in Figure 1A/B.

21. An isolated proton-gated cation channel comprising a subunit according to Claim 20.

22. An isolated nucleic acid encoding a proton-gated cation channel subunit, wherein said nucleic acid encodes the amino acid sequence show in Figure 1A/B.

## Patentansprüche

1. Isolierter Protonen-gesteuerter ("proton-gated") Kationen-Kanal, umfassend eine Untereinheit, die die in Figur 1A/B gezeigte Aminosäure-Sequenz oder eine Variante dieser Sequenz, die mindestens 531 Aminosäuren und mindestens 85% Identität hiermit besitzt, aufweist, wobei der Protonen-gesteuerte ("proton-gated") Kationen-Kanal einen zweiphasigen Strom entfaltet und die langsame Komponente des zweiphasigen Stroms durch Amilorid inhibiert wird.

2. Kanal gemäß Anspruch 1, welcher ein homopolymerer Kanal ist.

3. Kanal gemäß Anspruch 1, welcher ein heteropolymerer Kanal ist.

4. Kanal gemäß Anspruch 3, der mindestens eine Untereinheit aufweist, welche zu der Degenerin/ENaC-Kanal-Superfamilie gehört.

5. Kanal gemäß Anspruch 3, der mindestens eine Untereinheit aufweist, welche zu der P2X ATP-gesteuerten Kanal-Familie gehört.

6. Kanal gemäß Anspruch 5, wobei die Untereinheit der P2X-Familie P2X2 ist.

7. Verwendung eines Kanals gemäß jedem beliebigen der Ansprüche 1 bis 6 zum Screening nach Verbindungen, die als Liganden für den Protonen-gesteuerten ("proton-gated") Kationen-Kanal geeignet sind.

8. Isolierte Nukleinsäure, kodierend für eine Untereinheit des Protonen-gesteuerten ("proton-gated") Kationen-Kanals, der die in Figur 1A/B gezeigte Aminosäure-Sequenz oder eine Variante dieser Sequenz, die mindestens 531 Aminosäuren und mindestens 85% Identität hiermit besitzt, aufweist, wobei der Protonen-gesteuerte ("proton-gated") Kationen-Kanal einen zweiphasigen Strom entfaltet und die langsame Komponente des zweiphasigen Stroms durch Amilorid inhibiert wird.

9. Nukleinsäure, kodierend für eine Untereinheit des Protonen-gesteuerten ("proton-gated") Kationen-Kanals, wie in Anspruch 1 definiert, und eine andere Untereinheit des Kationen-Kanals, wie in jedem beliebigen der Ansprüche 4 bis 6 definiert.

10. Rekombinanter Vektor, umfassend eine Nukleinsäure, wie in Anspruch 8 oder 9 definiert.

11. Verfahren zum Herstellen eines Protonen-gesteuerten ("proton-gated") Kationen-Kanals gemäß jedem beliebigen der Ansprüche 1 bis 6, umfassend das Exprimieren einer Nukleinsäure-Sequenz gemäß Anspruch 8 oder 9, wobei das Verfahren kein Verfahren zur Behandlung des Körpers des Menschen oder eines Lebewesens durch Chirurgie oder Therapie bzw. kein diagnostisches Verfahren, das an dem Körper des Menschen oder eines Lebewesens angewendet wird, ist.

12. Rekombinante Wirtszelle, umfassend einen Vektor gemäß Anspruch 10, welcher in der Lage ist, den Kationen-Kanal gemäß Anspruch 1 oder 2 zu exprimieren, wobei die Wirtszelle im menschlichen Körper nicht vorhanden ist.

13. Rekombinante Wirtszelle, umfassend einen Vektor gemäß Anspruch 10, welcher in der Lage ist, den Kationen-Kanal gemäß jedem beliebigen der Ansprüche 3 bis 6 zu exprimieren, wobei die Wirtszelle im menschlichen Körper nicht vorhanden ist.

14. Verfahren zum Herstellen eines Protonen-gesteuerten ("proton-gated") Kationen-Kanals gemäß jedem beliebigen der Ansprüche 1 bis 6, umfassend das Inkubieren der rekombinanten Wirtszelle von Anspruch 12 oder 13 unter Bedingungen, welche die Expression der Nukleinsäure-Sequenz gestatten, und das Isolieren des Proteins aus der rekombinanten Wirtszelle.

15. Verwendung einer rekombinanten Wirtszelle gemäß Anspruch 13 oder 14 zum Screening nach Verbindungen, die als Liganden für den Protonen-gesteuerten ("proton-gated") Kationen-Kanal geeignet sind.

16. Verwendung einer Nukleinsäure, kodierend für einen Kanal gemäß jedem beliebigen der Ansprüche 1 bis 6, zum Screening nach Verbindungen, die als Liganden für den Protonen-gesteuerten ("proton-gated") Kationen-Kanal geeignet sind.

17. Zusammensetzung oder Kit zum Screening nach Verbindungen, die als Liganden für den Protonen-gesteuerten ("proton-gated") Kationen-Kanal geeignet sind, welche/welches mindestens einen Gegenstand, ausgewählt aus einem Kanal gemäß jedem beliebigen der Ansprüche 1 bis 6, einer Nukleinsäure gemäß Anspruch 8 oder 9, einem rekombinanten Vektor gemäß Anspruch 10, einer rekombinanten Wirtszelle gemäß Anspruch 12 oder 13, und jede beliebige geeignete hilfreiche Komponente umfasst.

18. Zusammensetzung oder Kit gemäß Anspruch 17, wobei die Liganden Analgetika sind.

19. Isolierter humaner Protonen-gesteuerter ("proton-gated") Kationen-Kanal, umfassend eine Untereinheit, die eine Aminosäure-Sequenz umfasst, die zu mindestens 85% mit der in Figur 1A/B gezeigten Aminosäure-Sequenz identisch ist, wobei der Protonen-gesteuerte ("proton-gated") Kationen-Kanal einen zweiphasigen Strom entfaltet, wenn er durch eine extrazelluläre Protonen-Konzentration aktiviert wird, welche unter dem physiologischen pH liegt, und wobei die langsame Komponente des zweiphasigen Stroms durch Amilorid inhibiert wird.

20. Isolierte Untereinheit des Protonen-gesteuerten ("proton-gated") Kationen-Kanals, bestehend aus der in Figur 1A/B gezeigten Aminosäure-Sequenz.

21. Isolierter Protonen-gesteuerter ("proton-gated") Kationen-Kanal, umfassend eine Untereinheit gemäß Anspruch 20.

22. Isolierte Nukleinsäure, kodierend für eine Untereinheit des Protonen-gesteuerten ("proton-gated") Kationen-Kanals, wobei die Nukleinsäure für die in Figur 1A/B gezeigte Aminosäure-Sequenz kodiert.

## Revendications

1. Canal de cations activable par les protons isolé comprenant une sous-unité ayant la séquence d'acides aminés représentée sur la Figure 1A/B ou un variant de cette séquence ayant au moins 531 acides aminés et au moins 85 % d'identité avec elle, où le canal de cations activable par les protons présente un courant biphasique et la composante lente du courant biphasique est inhibée par l'amiloride.

2. Canal selon la revendication 1, qui est un canal homopolymère.

3. Canal selon la revendication 1, qui est un canal hétéropolymère.

4. Canal selon la revendication 3 ayant au moins une sous-unité qui appartient à la superfamille de canaux dégénérine/ENaC.

5. Canal selon la revendication 3 ayant au moins une sous-unité qui appartient à la famille des récepteurs canaux P2X de l'ATP.

6. Canal selon la revendication 5, dans lequel la sous-unité de la famille P2X est P2X2.

7. Utilisation d'un canal selon l'une quelconque des revendications 1 à 6 pour cribler des composés utiles comme ligands de canal de cations activable par les protons.

8. Acide nucléique isolé codant une sous-unité de canal de cations activable par les protons ayant la séquence d'acides aminés représentée sur la Figure 1A/B ou un variant de cette séquence ayant au moins 531 acides aminés et au moins 85 % d'identité avec elle, où le canal de cations activable par les protons présente un courant biphasique et la composante lente du courant biphasique est inhibée par l'amiloride.

9. Acide nucléique codant une sous-unité de canal de cations activable par les protons telle que définie dans la revendication 1 et une autre sous-unité de canal de cations telle que définie dans l'une quelconque des revendications 4 à 6.

10. Vecteur recombinant comprenant un acide nucléique tel que défini dans la revendication 8 ou 9.

11. Procédé de production d'un canal de cations activable par les protons selon l'une quelconque des revendications 1 à 6 consistant à exprimer une séquence d'acide nucléique selon la revendication 8 ou 9, où ledit procédé n'est pas un procédé pour le traitement du corps humain ou animal par chirurgie ou thérapie ou un procédé de diagnostic pratiqué sur le corps humain ou animal.

12. Cellule hôte recombinante comprenant un vecteur selon la revendication 10 qui est capable d'exprimer le canal de cations selon la revendication 1 ou 2, ladite cellule hôte n'étant pas présente dans le corps humain.

13. Cellule hôte recombinante comprenant un vecteur selon la revendication 10 qui est capable d'exprimer le canal de cations selon l'une quelconque des revendications 3 à 6, ladite cellule hôte n'étant pas présente dans le corps humain.

14. Procédé de production d'un canal de cations activable par les protons selon l'une quelconque des revendications 1 à 6 comprenant l'incubation de la cellule hôte recombinante de la revendication 12 ou 13 dans des conditions qui permettent à la séquence d'acide nucléique d'être exprimée, et l'isolement de la protéine à partir de la cellule hôte recombinante.

15. Utilisation d'une cellule hôte recombinante selon la revendication 13 ou 14 pour cribler des composés utiles comme ligands de canal de cations activable par les protons.

16. Utilisation d'un acide nucléique codant un canal selon l'une quelconque des revendications 1 à 6 pour cribler des composés utiles comme ligands de canal de cations activable par les protons.

17. Composition ou trousse pour cribler des composés utiles comme ligands de canal de cations activable par les protons, qui comprend au moins un parmi un canal selon l'une quelconque des revendications 1 à 6, un acide nucléique selon la revendication 8 ou 9, un vecteur recombinant selon la revendication 10, une cellule recombinante selon la revendication 12 ou 13, et tout composant auxiliaire approprié.

18. Composition ou trousse selon la revendication 17 dans laquelle les ligands sont des analgésiques.

19. Canal de cations activable par les protons isolé comprenant une sous-unité qui comprend une séquence d'acides aminés qui est au moins identique à 85 % à la séquence d'acides aminés représentée sur la Figure 1A/B, où le canal de cations activable par les protons présente un courant biphasique lorsqu'il est activé par une concentration de protons extracellulaire qui est inférieure au pH physiologique, et dans lequel la composante lente du courant biphasique est inhibée par l'amiloride.

20. Sous-unité de canal de cations activable par les protons isolé constituée de la séquence d'acides aminés représentée sur la Figure 1A/B.

21. Canal de cations activable par les protons isolé comprenant une sous-unité selon la revendication 20.

22. Acide nucléique isolé codant une sous-unité de canal de cations activable par les protons, ledit acide nucléique codant la séquence d'acides aminés représentée sur la Figure 1A/B.


### SEQUENCE LISTING

(1) GENERAL INFORMATION:
   (i) APPLICANT:
      (A) NAME: McGill University
      (B) STREET: 3550 University Street
      (C) CITY: Montreal
      (D) STATE: Quebec
      (E) COUNTRY: CANADA
      (F) POSTAL CODE (ZIP): H3A 2A7
      (G) TELEPHONE: (514) 398-9898
      (H) TELEFAX: (514) 398-8479

      (A) NAME: SEGUELA, Philippe
      (B) STREET: 890 Davaar
      (C) CITY: Outremont
      (D) STATE: Quebec
      (E) COUNTRY: CANADA
      (F) POSTAL CODE (ZIP): H2V 3B5

      (A) NAME: BABINSKI, Kazimierz
      (B) STREET: 364 Berkley Circle
      (C) CITY: Dorval
      (D) STATE: Quebec
      (E) COUNTRY: CANADA
      (F) POSTAL CODE (ZIP): H9S 1H4
   (ii) TITLE OF INVENTION: DNA ENCODING A HUMAN PROTON-GATED ION CHANNEL AND USES THREOF
   (iii) NUMBER OF SEQUENCES: 5
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30 (EPO)
   (v) CURRENT APPLICATION DATA:
      APPLICATION NUMBER: CA PCT/CA98/01016
   (vi) PRIOR APPLICATION DATA:
      (A) APPLICATION NUMBER: CA 2,219,713
      (B) FILING DATE: 29-OCT-1997
(2) INFORMATION FOR SEQ ID NO: 1:
   (i) SEQUENCE CHARACTERISTICS:
      (A) LENGTH: 1732 base pairs
      (B) TYPE: nucleic acid
      (C) STRANDEDNESS: double
      (D) TOPOLOGY: linear
   (ii) MOLECULE TYPE: cDNA
   (ix) FEATURE:
      (A) NAME/KEY: CDS
      (B) LOCATION:22..1614
   (xi) SEQUENCE DESCRIPTION: SEQ ID NO: 1:
   TCGCACGACG CGGTTCTGGC C ATG AAG CCC ACC TCA GGC CCA GAG GAG GCC        51
   CGG CGG CAG CCC TCG GAC ATC CGC GTG TTC GCC AGC AAC TGC TCG ATG        99
   CAC GGG CTG GGC CAC GTC TTC GGG CCA GGC AGC CTG AGC CTG CGC CGG       147
   GGG ATG TGG GCA GCG GCC GTG GTC CTG TCA GTG GCC ACC TTC CTC TAC       195
   CAG GTG GCT GAG AGG GTG CGC TAC TAC AGG GAG TTC CAC CAC CAG ACT       243
   GCC CTG GAT GAG CGA GAA AGC CAC CGG CTC GTC TTC CCG GCT GTC ACC       291
   CTG TGC AAC ATC AAC CCA CTG CGC CGC TCG CGC CTA ACG CCC AAC GAC       339
   CTG CAC TGG GCT GGG TCT GCG CTG CTG GGC CTG GAT CCC GCA GAG CAC       387
   GCC GCC TTC CTG CGC GCC CTG GGC CGG CCC CCT GCA CCG CCC GGC TTC       435
   ATG CCC AGT CCC ACC TTT GAC ATG GCG CAA CTC TAT GCC CGT GCT GGG       483
   CAC TCC CTG GAT GAC ATG CTG CTG GAC TGT CGC TTC CGT GGC CAA CCT       531
   TGT GGG CCT GAG AAC TTC ACC ACG ATC TTC ACC CGG ATG GGA AAG TGC       579
   TAC ACA TTT AAC TCT GGC GCT GAT GGG GCA GAG CTG CTC ACC ACT ACT       627
   AGG GGT GGC ATG GGC AAT GGG CTG GAC ATC ATG CTG GAC GTG CAG CAG      675
   GAG GAA TAT CTA CCT GTG TGG AGG GAC AAT GAG GAG ACC CCG TTT GAG      723
   GTG GGG ATC CGA GTG CAG ATC CAC AGC CAG GAG GAG CCG CCC ATC ATC      771
   GAT CAG CTG GGC TTG GGG GTG TCC CCG GGC TAC CAG ACC TTT GTT TCT      819
   TGC CAG CAG CAG CAG CTG AGC TTC CTG CCA CCG CCC TGG GGC GAT TGC      867
   AGT TCA GCA TCT CTG AAC CCC AAC TAT GAG CCA GAG CCC TCT GAT CCC      915
   CTA GGC TCC CCC AGC CCC AGC CCC AGC CCT CCC TAT ACC CTT ATG GGG      963
   TGT CGC CTG GCC TGC GAA ACC CGC TAC GTG GCT CGG AAG TGC GGC TGC     1011
   CGA ATG GTG TAC ATG CCA GGC GAC GTG CCA GTG TGC AGC CCC CAG CAG     1059
   TAC AAG AAC TGT GCC CAC CCG GCC ATA GAT GCC ATC CTT CGC AAG GAC     1107
   TCG TGC GCC TGC CCC AAC CCG TGC GCC AGC ACG CGC TAC GCC AAG GAG     1155
   CTC TCC ATG GTG CGG ATC CCG AGC CGC GCC GCC GCG CGC TTC CTG GCC     1203
   CGG AAG CTC AAC CGC AGC GAG GCC TAC ATC GCG GAG AAC GTG CTG GCC     1251
   CTG GAC ATC TTC TTT GAG GCC CTC AAC TAT GAG ACC GTG GAG CAG AAG     1299
   AAG GCC TAT GAG ATG TCA GAG CTG CTT GGT GAC ATT GGG GGC CAG ATG     1347
   GGC CTT TTC ATC GGG GCC AGC CTG CTC ACC ATC CTC GAG ATC CTA GAC      1395
   TAC CTC TGT GAG GTG TTC CGA GAC AAG GTC CTG GGA TAT TTC TGG AAC      1443
   CGA CAG CAC TCC CAA AGG CAC TCC AGC ACC AAT CTG CTT CAG GAA GGG      1491
   CTG GGC AGC CAT CGA ACC CAA GTT CCC CAC CTC AGC CTG GGC CCC AGA      1539
   CCT CCC ACC CCT CCC TGT GCC GTC ACC AAG ACT CTC TCC GCC TCC CAC      1587
   CGC ACC TGC TAC CTT GTC ACA CAG CTC TAGACCTGCT GTCTGTGTCC            1634
   TCGGAGCCCC GCCCTGACAT CCTGGACATG CCTAGCCTGC ACGTAGCTTT TCCGTCTTCA    1694
   CCCCAAATAA AGTCCTAATG CATCAAAAAA AAAAAAAA                            1732
(2) INFORMATION FOR SEQ ID NO: 2:
   (i) SEQUENCE CHARACTERISTICS:
      (A) LENGTH: 531 amino acids
      (B) TYPE: amino acid
      (D) TOPOLOGY: linear
   (ii) MOLECULE TYPE: protein
   (xi) SEQUENCE DESCRIPTION: SEQ ID NO: 2:
(2) INFORMATION FOR SEQ ID NO: 3:
   (i) SEQUENCE CHARACTERISTICS:
      (A) LENGTH: 17 amino acids
      (B) TYPE: amino acid
      (C) STRANDEDNESS: single
      (D) TOPOLOGY: linear
   (ii) MOLECULE TYPE: protein
   (ix) FEATURE:
      (A) NAME/KEY: Region
      (B) LOCATION:2
      (D) OTHER INFORMATION:/note= "Xaa = unidentified amino acid."
   (ix) FEATURE:
      (A) NAME/KEY: Region
      (B) LOCATION:11
      (D) OTHER INFORMATION:/note= "Xaa = unidentified amino acid."
   (ix) FEATURE:
      (A) NAME/KEY: Region
      (B) LOCATION:13..14
      (D) OTHER INFORMATION:/note= "Xaa = unidentified amino acid."
   (ix) FEATURE:
      (A) NAME/KEY: Region
      (B) LOCATION:16
      (D) OTHER INFORMATION:/note= "Xaa = unidentified amino acid."
   (xi) SEQUENCE DESCRIPTION: SEQ ID NO: 3:
   Leu Xaa Phe Pro Ala Val Thr Leu Cys Asn Xaa Asn Xaa Xaa Arg Xaa
   1               5                   10                  15
   Ser
(2) INFORMATION FOR SEQ ID NO: 4:
   (i) SEQUENCE CHARACTERISTICS:
      (A) LENGTH: 20 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 4:
      TCAGTGGCCA CCTTCCTCTA 20
(2) INFORMATION FOR SEQ ID NO: 5:
   (i) SEQUENCE CHARACTERISTICS:
      (A) LENGTH: 21 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 5:
      ACAGTCCAGC AGCATGTCAT C 21